# EUROPÄISCHE PATENTANMELDUNG

(11) **EP 0 743 074 A2**
(43) Veröffentlichungstag der Anmeldung: **20.11.1996**
(21) Anmeldenummer: 96105914.4
(22) Anmeldetag: 16.04.1996
(51) Int. Cl.: A61M 15/00, A61M 16/06

(54) **Atemschutzmaske**

(30) Priorität: 18.05.1995 DE 29508234 U
(71) Anmelder: Ruther, Hans Martin, 88145 Opfenbach (DE)
(72) Erfinder: Ruther, Hans Martin, 88145 Opfenbach (DE)
(74) Vertreter: Riebling, Peter, Dr.-Ing.

(57) **Zusammenfassung**

Beschreiben wird eine Maske zum Inhalieren von verschiedenen Wirkstoffen, wobei die Maske aus einem Grundkörper und einer daran angebrachten Abdeckung besteht, wobei zwischen diese Teile ein mit Wirkstoffen getränktes Pad eingelegt ist.

## Beschreibung

Gegenstand der vorliegenden Erfindung ist eine Maske nach dem Oberbegriff des Anspruchs 1.

Eine ähnliche Maske ist durch das auf den gleichen Anmelder zurückgehende Gebrauchsmuster G 89 06 445.3 bekannt geworden.

Hier war eine einstückige und nur einmal verwendbare Maske vorgesehen, die mit dem jeweiligen, gewünschten Wirkstoff von vornherein getränkt war.

Nachteilig bei dieser Maske ist, daß diese nur einmal verwendbar ist. Nach der Verwendung musste stets die gesamte Maske weggeworfen und entsorgt werden. Dies führt nicht nur zu Kostenproblemen, sondern auch zu einer unnötigen Belastung der Umwelt.

Aufgabe der vorliegenden Erfindung ist es daher, eine Maske bereit zu stellen, die mit einfachen Mitteln kostengünstiger und wiederverwendbar gestaltet werden kann.

Erfindungsgemäss wird diese Aufgabe durch die technische Lehre des kennzeichnenden Teils von Anspruch 1 gelöst.

Hierbei ist wesentlich, daß nun ein Grundkörper mit einer daran angebrachten Abdeckung vorgesehen ist. In den Zwischenraum zwischen dem Grundkörper und der Abdeckung kann ein mit Wirkstoffen getränktes Pad eingesetzt werden.

Vorteilhaft hierbei ist, daß nun nicht mehr nach dem Verbrauch der Wirkstoffe die gesamte Maske weggeworfen werden muß, sondern es genügt, das Pad auszuwechseln.

Ein weiterer Vorteil ist, daß durch die Auswechselbarkeit der Pads nunmehr eine Maske mit verschiedenen Wirkstoffen versehen werden kann. Je nach den Wünschen des Benutzers bzw. den medizinischen Erfordernissen können einfach die erforderlichen Pads gekauft und in die Maske eingelegt werden. Der Erwerb einer neuen Maske ist hierzu nicht erforderlich.

Die Abdeckung ist hierbei bevorzugt drehbar am Grundkörper befestigt und wird in der geschlossenen Stellung durch einen Schnappverschluß gehalten.

Zur Befestigung der Maske am Kopf des Benutzers sind an der Abdeckung Laschen vorgesehen, wobei in diese Laschen ein entsprechendes Spannband eingesetzt oder in anderer Weise befestigt werden kann. Das Spannband selbst wird dann um den Kopf des Benutzers geführt und an der gegenüberliegenden Lasche der Abdeckung befestigt.

Es ist selbstverständlich ebenfalls möglich, das Spannband am Grundkörper direkt zu befestigen. Alternativ ist es auch möglich, zwei Spannbänder zu verwenden, die dann hinter dem Kopf des Benutzers verknotet werden.

In vorteilhafter Weise ist die Maske an die Gesichtsform des Benutzers angepaßt. Um Druckstellen im Gesicht zu verhindern, ist weiterhin vorgesehen, die Anlagefläche des Grundkörpers bzw. der Maske mit einem weichen Material zu überdecken. Dieses Material kann beispielsweise Stoff oder Schaumgummi sein.

Ein weiterer wesentlicher Vorteil der Maske ist, daß sie zerlegt, das Spannband und die Polsterung am Grundkörper abgenommen und jedes der Elemente für sich einzeln gereinigt werden kann. Die Maske kann hierbei nach Entfernen der Anbauteile ohne Probleme in eine handelsübliche Spülmaschine eingelegt und dort gereinigt werden. Das Spannband sowie die Auflage am Grundkörper können in der Waschmaschine gewaschen werden.

Die Pads selbst sind aus Stoff, Baumwolle, Vliesmaterial oder dergleichen hergestellt. Es ist selbstverständlich ebenfalls möglich, bei der Verwendung von geeigneten Pads diese ebenfalls nach der Benutzung zu waschen und danach erneut zu verwenden.

Als Wirkstoffe für die neuerungsgemässe Maske können Erkältungsmittel zum Einsatz kommen, Mittel gegen Stirnhöhlenentzündungen, anderen entzündungshemmende oder die Schleimhaut beruhigende Stoffe sowie weitere Stoffe zur Behandlung von Asthmatikern oder ähnlich Bronchialkranken.

Es ist selbstverständlich ebenfalls möglich, Stoffe, die keine erwiesene medizinische Wirkung haben, aber ein subjektives Wohlbefinden auslösen, zu verwenden.

Dies kann beispielsweise im Rahmen einer Aromatherapie geschehen.

Die Maske selbst kann in verschiedenen Größen, Formen und Farbgebungen ausgebildet sein. Insbesondere bei der Verwendung der Maske zur Behandlung von Kindern können hierbei verschiedene bekannte Comic-Gestalten nachgebildet oder ein peppiges Design verwendet werden.

Ein weiterer wesentlicher Vorteil der Maske ist, daß nach dem Aufsetzen praktisch keine Behinderung vorliegt. Dies Wahrnehmung wird durch die Maske nicht im geringsten eingeschränkt, so daß Fernsehen, Lesen oder andere Tätigkeiten ohne Probleme wahrgenommen werden können. Bei entsprechend deutlicher Aussprache ist es ebenfalls möglich, die Maske in der Arbeit zu verwenden, da der Träger immer noch verstanden wird.

Der Erfindungsgegenstand der vorliegenden Neuerung ergibt sich nicht nur aus dem Gegenstand der einzelnen Schutzansprüche, sondern auch aus der Kombination der einzelnen Schutzansprüche untereinander.

Alle in den Unterlagen, einschließlich der Zusammenfassung, offenbarten Angaben und Merkmale, insbesondere die in den Zeichnungen dargestellte räumliche Ausbildung werden als erfindungswesentlich beansprucht, soweit sie einzeln oder in Kombination gegenüber dem Stand der Technik neu sind.

Im folgenden wird die Erfindung anhand von mehrere Ausführungswege darstellenden Zeichnungen näher erläutert. Hierbei gehen aus den Zeichnungen und ihrer Beschreibung weitere erfindungswesentliche Merkmale und Vorteile der Erfindung hervor.

Dabei zeigt:
- Figur 1:: eine Seitenansicht der neuerungsgemäßen Maske,
- Figur 2:: eine Draufsicht in Pfeilrichtung 2 auf den Grundkörper.

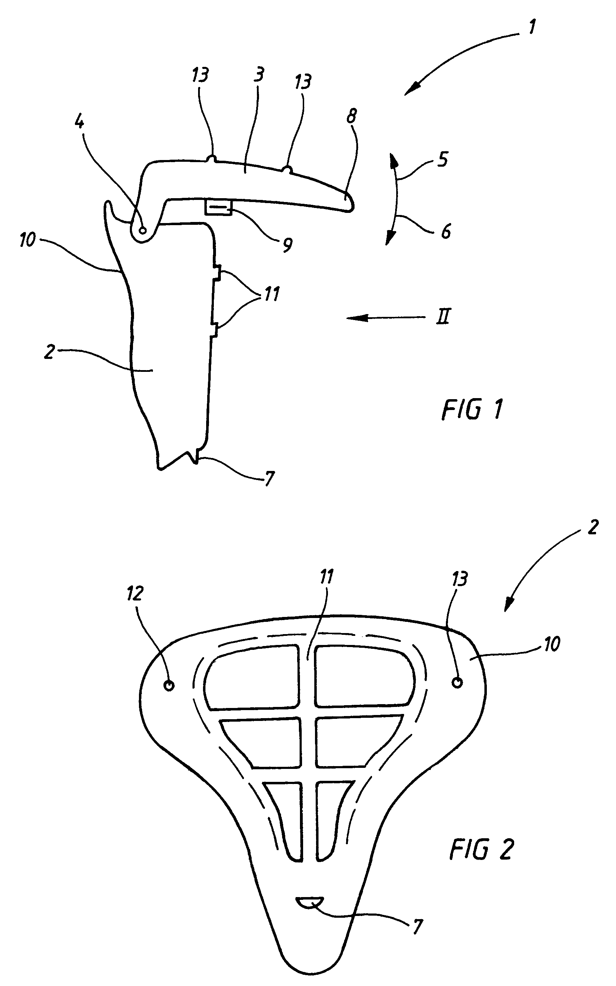

Die Maske 1 besteht gemäß Figur 1 aus einem Grundkörper 2, an dem eine Abdeckung 3 befestigt ist. Die Befestigung erfolgt hierbei an der Position 4, so daß die Abdeckung selbst in Pfeilrichtung 5,6 verschwenkbar ist.

Wird die Abdeckung 3 vollständig in Pfeilrichtung 6 verschwenkt, so übergreift sie mit ihrem vorderen Ende 8 einen am Grundkörper angebrachten Vorsprung 7, so daß sich insgesamt ein Schnappverschluß ergibt.

Selbstverständlich ist auch die kinematische Umkehr dieses Schnappverschlußes möglich.

An der Abdeckung 3 sind Laschen 9 angebracht, die zur Befestigung von nicht näher dargestellten Spannbändern dienen.

Der Grundkörper weist an seiner dem Gesicht des Benutzers zugewandten Seite eine Anlagefläche 10 auf, die in der Formgebung etwa der Form eines Gesichts angepasst ist. Diese Anlagefläche kann mit einem nicht näher dargestellten Stoffstück oder einem anderen weichen bzw. federnden Material überzogen werden, um Druckstellen im Gesicht des Benutzers zu vermeiden.

Sowohl der Grundkörper 2 als auch die Abdeckung 3 weisen Gitter 11,13 auf.

Zur Verwendung der Maske wird nun ein nicht näher dargestelltes Pad, das in seiner Form entsprechend an die Form des Grundkörpers 2 bzw. der Abdeckung 3 angepaßt ist, auf das Gitter 11 des Grundkörpers 2 aufgelegt. Danach wird die Abdeckung 3 in Pfeilrichtung 6 geschlossen und eingerastet. Die Maske ist dann verwendungsfähig und muß nur noch aufgesetzt werden.

Figur 2 zeigt eine Draufsicht auf den Grundkörper 2. Hier wird deutlich, daß die Abdeckung 3 bevorzugt mit entsprechenden Achsen Ausnehmungen 12 am Grundkörper 2 durchgreift und somit insgesamt drehbar bzw. schwenkbar gelagert ist.

Zum Wechseln des Pads wird die Abdeckung 3 in Pfeilrichtung 5 nach dem Lösen des Schnappverschlußes hochgeklappt, das alte Pad wird entnommen und ggf. ein neues Pad eingesetzt.

Da die gesamte Maske 1 bevorzugt aus Plastik hergestellt ist und somit die Einzelteile 2,3 eine gewisse Elastizität aufweisen, ist das Lösen des Schnappverschlußes kein Problem.

Insgesamt ergibt sich somit bei längerfristiger Verwendung der Maske eine Kostenentlastung sowie ein positiver Effekt für die Umwelt.

### Zeichenlegende

- 1: Maske
- 2: Grundkörper
- 3: Abdeckung
- 4: Position
- 5: Pfeilrichtung
- 6: "
- 7: Vorsprung
- 8: Ende
- 9: Lasche
- 10: Anlagefläche
- 11: Gitter
- 12: Ausnehmung
- 13: Gitter

## Patentansprüche

1. Maske zum Inhalieren von verschiedenen Wirkstoffen, **dadurch gekennzeichnet**, daß die Maske (1) aus einem Grundkörper (2) und einer daran angebrachten Abdeckung (3) besteht, wobei zwischen diese Teile (2,3) ein mit Wirkstoffen getränktes Pad eingelegt ist.

2. Maske nach Anspruch 1, **dadurch gekennzeichnet**, daß die Abdeckung (3) drehbar am Grundkörper (2) befestigt ist.

3. Maske nach Anspruch 1 oder 2, **dadurch gekennzeichnet**, daß zum Fixieren der Abdeckung (3) in einer geschlossenen Stellung ein Schnappverschluß vorgesehen ist.

4. Maske nach einem der Ansprüche 1 bis 3, **dadurch gekennzeichnet**, daß die Abdeckung (3) mit Laschen (9) zur Befestigung eines Spannbandes versehen ist.

5. Maske nach einem der Ansprüche 1 bis 4, **dadurch gekennzeichnet**, daß die Maske (1) an die Gesichtsform angepasst ist.

6. Maske nach einem der Ansprüche 1 bis 5, **dadurch gekennzeichnet,** daß die Anlagefläche (10) des Grundkörpers (2) durch ein weiches Material bedeckt ist.
